Europäisches Patentamt

European Patent Office

Office européen des brevets

(11) Publication number : **0 508 963 A1**

# (12) EUROPEAN PATENT APPLICATION

(21) Application number : 92830159.7

(22) Date of filing : 02.04.92

(51) Int. Cl.⁵ : **A61B 5/103**, A43D 1/02

(30) Priority : **12.04.91 IT FI910082**

(43) Date of publication of application :
**14.10.92 Bulletin 92/42**

(84) Designated Contracting States :
**AT BE CH DE DK ES FR GB GR LI LU MC NL PT SE**

(71) Applicant : **PERSONAL SHOES S.p.A.**
**Via Agnolo Poliziano No.8**
**I-50129 Firenze (IT)**

(72) Inventor : **Galluzzo, Paolino**
**Via Barberinese n. 207**
**I-50013 Campi Bisenzio, Firenze (IT)**

(74) Representative : **Mannucci, Gianfranco,**
**Dott.-Ing. et al**
**Ufficio Tecnico Ing. A. Mannucci Via della**
**Scala 4**
**I-50123 Firenze (IT)**

(54) **Method and apparatus for making customised plantar templates.**

(57)   A method for making plantar templates comprises the following steps :
detecting the distribution of the foot load on a footboard (3) ;
recording and processing data relevant to the load distribution ;
transmitting the processed data to a numerically controlled machine tool (25), which works a block of material (27) for obtaining a plantar template whose shape is determined according to the conformation of the user's foot.

Fig. 1

EP 0 508 963 A1

The invention refers to a method and apparatus for making plantar templates suited to the conformation of the foot of any single user.

Currently, in particular for the correction of foot pathologies, corrective arch supports of standard type, i.e.of non custom-tailored type, are used which are chosen in relation to the defect to be corrected or to the pathology the user suffers from. Alternatively, customised plantar templates are made on the basis of data obtained from suitable apparatus. These apparatus include a footboard provided with sensors which detect the pattern and distribution of the foot loads, and are able to process the acquired data to yield charts and diagrams which visualize the pattern and distribution of the loads. The technician must then, on the basis of this information, manually shape a plantar template suited to the user's foot. This type of working is extremely costly and time consuming, and requires handicraft operations. Though justified in case of medical prescription for the correction of serious pathologies, it cannot be used (owing its high costs) for making custom-tailored plantar templates which have the only object to make the deambulation or, in some particular cases, the sports activity, more comfortable.

It is therefore an object of the invention to provide a method and relevant apparatus or system which allow the production of customised plantar templates i.e. suited to the conformation of the foot of every single user) with relatively limited costs and in short times.

Substantially, the method according to the invention is characterized by the following steps:
– detecting the distribution of the foot load on the ground or on a footboard;
– storing and processing data relevant to the load distribution;
– using the processed data for controlling a machine tool which works a block of material to obtain therefrom a plantar template whose shape depends on the conformation of the user's foot and on the load distribution previously assessed.

To make a plantar template allowing a comfortable deambulation, it is advantageous that the detection of data relevant to the distribution of loads be provided both in static and dynamic conditions, that is, during the deambulation. Moreover, provision may also be made for information relevant to the anatomy, shape and dimensions of the foot to be added to those concerning the distribution of the loads, so as to allow for a correct choice of the shoes within which the template - which is made on the basis of the information relevant to the loads distribution - must be introduced. The assessment of the shape, dimension and anatomy of the user's foot makes it possible to select a shoe chosen from a limited number of standard shapes, according to the ratios between width, height and lenght of the foot.

Practically, by the method according to the invention, it is possible to carry out the detection of all the user's data at one seat, and then transfer these data to a device for the processing and management thereof to control a machine tool that carries out the working of the template. It is thus possible to carry out the detection at one seat and the working at another remote seat and, if required in a different point in time, which remote seat may be a shoe factory, or other suitable workshop, where the working operations on more pairs of plantar templates for different users are carried out in an automated manner, each pair of templates being custom made to suit the relevant user.

The apparatus for carrying out the above described method, comprises in practice: means for detecting the distribution of the foot loads exerted on the ground or a suitable footboard, means for storing the detected data, means for processing the data, and a numerical control machine tool for working 'out the plantar template on the basis of the processed data.

In an improved embodiment of the apparatus according to the invention, further means are provided for detecting the conformation of the foot and, in particular, the anatomy, dimension and shape thereof.

The invention will be better understood by the following description and accompanying drawing, which shows a practical, not limiting exemplification of the said invention. In the drawing:
Fig. 1 shows a schematic representation of the apparatus according to the invention;
Fig. 2 shows a detail of the means for assessing the foot shape; and
Figg. 3A and 3B show a block diagram of the method according to the invention.

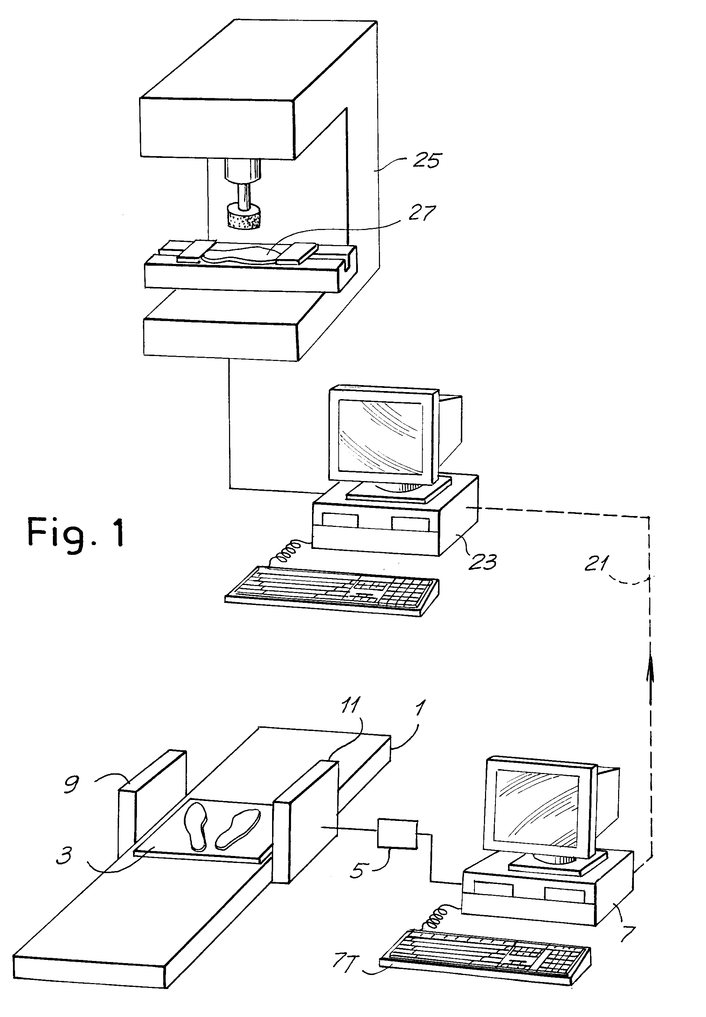

With initial reference to Fig. 1, an apparatus is provided which comprises a support 1 on which a footboard 3 is disposed which includes a plurality of sensors of for example, piezoelectric type, able to detect the distribution of the weight of a person who stands on the footboard 3 or who walks along the support 1 thereof. The sensors associated to the footboard 3 are connected via an interface 5 to an electronic processor 7, for example a personal computer. The equipment, consisting of the elements 3, 5 and 7, may be made on purpose or made up of a commercially available device. Particularly suitable for this application is the ORTHOMAT[R] marketed by Polysens S.p.A. (Tavarnelle Val di Pesa, Florence, Italy). This apparatus is normally used for the analysis of the foot conformation and for the graphic representation of the loads distribution which forms a basis for the podologist to make handicraft plantar template of currently known type. Within the scope of the method according to the present invention, the ORTHOMAT machine, or other equivalent, is employed according to procedures other than those originally provided for, and is used to carry out a fully automated working process for the production of custom-tailored plantar templates.

The footboard 3 may be made, for example, by means of a matrix of 1024 piezoelectric sensors of circular shape disposed in such a way as to cover a substantially square area having sides of 32 cm. These sensors are able to acquire the load exerted by the foot at each of the 1024 areas into which the 32x32 cm surface is divided. The processor 7 is so programmed as to perform a predetermined number of consecutive acquisitions at preset time intervals, with a preset scanning frequency. In practice, there may be provided 512 successive acquisitions of as many frames of data, at a scanning frequency varying from 12.5 to 100 frames per second, with a duration of data detection in the range of 40 to 5 seconds according to the preset frequency. Each acquired frame of data consists of values relevant to the loads acting on the 1024 sensors. For a successive correct working, the maximum load value on every single sensor is selected, and a so-called frame of peaks, that is, a set of maximum load values over the whole 1024 sensors provided on the footboard, is acquired. The acquisitions may be carried out under static and dynamic conditions. In particular, under dynamic conditions (that is, when the user is in motion) two series of acquisitions are carried out, one for the left foot, and the other for the right foot.

In a known manner, the management program for the detection of data may provide for various types of processing, in order to acquire data relevant to both static and dynamic conditions. In particular, several fixed representations of acquired data may be made, particularly in relation to:

– the configuration and surface of the contact area between foot and ground;
– the space distribution of loads in plan and perspective view, and the unit value of loads on each sensor;
– the time pattern of the load weighing upon every single sensor;
– the calculation of the pressure centre.

Processings may also be made of data relevant to dynamic conditions to determine, for example, the variation of the contact area during time or the displacement of the pressure centre, the displacement of the point of maximum load, and so on. The detection of data for the study of the dynamic effects may be carried out through sequential acquisition of data from a person walking along the support 1, by moving one foot after the other onto the footboard 3.

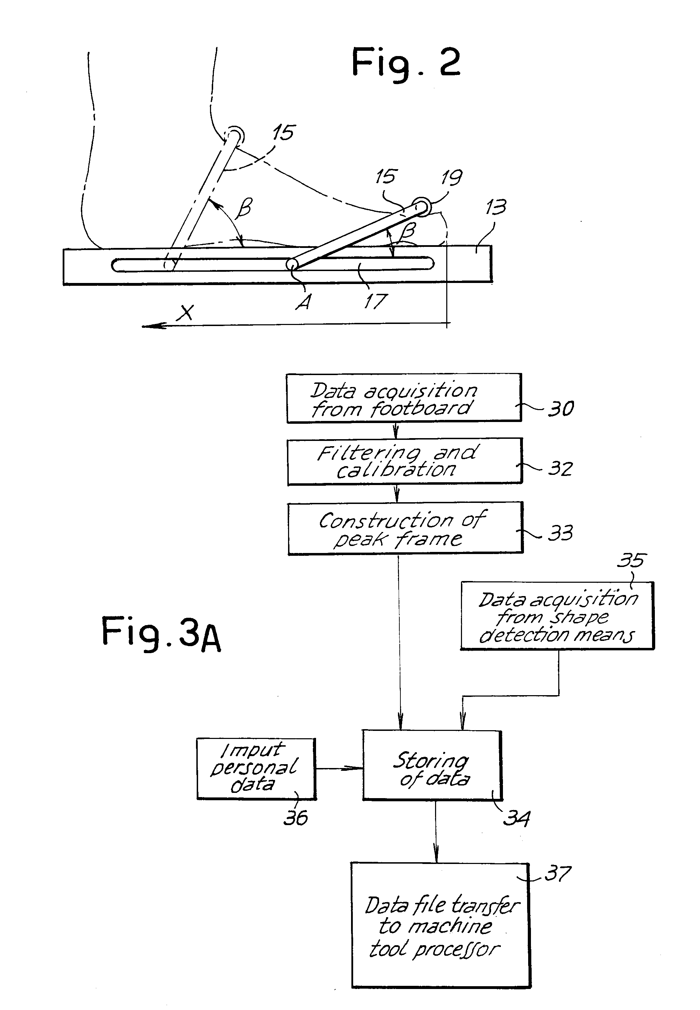

The detection system is completed by sensor means of electromechanic or electronic type, capable of detecting the foot profile and thus the shape and dimensions thereof. In fact, the footboard 3 is able to exclusively detect the distribution of the loads and the plan shape of the foot, but does not provide data concerning the tridimensional shape of the foot. In Fig. 1 the foot shape-detecting means are schematically indicated at 9 and 11, while a possible electromechanic embodiment thereof is indicated in Fig. 2. In the electronic embodiment, these detection means may consist of a matrix or array of photoemitters and a corresponding matrix and array of photosensors disposed at the sides of the detection region defined by the footboard 3. The photoemitters emit beams of light which are detected by the photosensors. The photosensors are interfaced to the processor 7 and, depending on whether the light signal is present or not thereon, the corresponding signal emitted by each photosensor will make it possible to reconstruct the side shape of the foot.

In the electromechanic embodiment of Fig. 2, a support 13 and a bracket 15 are provided, able to translate along an axis X - defined by a guide 17 - and to rotate about an axis A movable along the guide 17. The bracket 15 carries a roller 19 which is made to rest on the back of the foot. The detection of the foot shape and dimension is carried out by rolling the roller 19 on the back of the foot and sensing the positions taken up in succession by the axis A along the guide 17 and the angular positions defined by the angle β of bracket 15 as well. The detected data are then digitized and sent to the computer 7.

In more sophisticated embodiments for the detection of the foot shape and dimension, provision may be made for a telecamera located sideway of the footboard 3 and interfaced to the computer 7, or to optical scanning means. An image-processing software, of a type known per se, may be used to obtain, from the images detected by the telecamera, the data relevant to the shape and dimension of the foot under examination.

Moreover, as the load sensors detect only the regions of contact between the foot and the ground and, therefore, do not provide a projection of the complete sole of the foot, it may be convenient to provide optical scanning means to detect the shape of the sole of the foot. These optical scanning (or equivalent) means may be disposed, for example, under a further, transparent footboard 3, below which the scanning means are disposed.

Whatever the type of means employed for the detection of the foot shape, dimension and anatomy, the software program which control the data detection is able to acquire both data obtained from the sensors of footboard 3 under static and dynamic conditions, and data relevant to the foot outline for determining the maximum height at the instep and other parameters necessary to define the whole shape of the limb and, consequently, the size and type of shoe (short, large, and so on) that must be selected and associated to the plantar template to be produced, according to the data sensed by the footboard 3, in a manner described below.

The data detected through the footboard 3 (and possibly through means 9, 11 for the detection of the foot dimension and shape and/or through the scanning means for the detection of the plan projection) are either recorded on a suitable magnetic support or transmitted via modem through a transmission line 21 to a second electronic processor which controls a machine tool 25 of a type known per se. The processor 23 can be the same microprocessor of machine 25. In any case, the processor 23 is programmed by a software program of CAD-CAM type which works on data coming from the processor 7. In particular, data relevant to the frame of peaks are processed by the CAD-CAM software for controlling the machine 25 which works a block 27 of plastic material or other material suitable for this application, to produce the shaped plantar template.

Data transmitted by the processor 7 to the processor 23 may include personal data or other information entered through the keyboard 7T of processor 7, and which remain associated to the data relevant to the loads distribution and to the shape of the foot; in this way, within the department where the machine tool 25 is located, it is possible to easily determine for which user the plantar templates are, which plantar templates are produced by the machine tool 25.

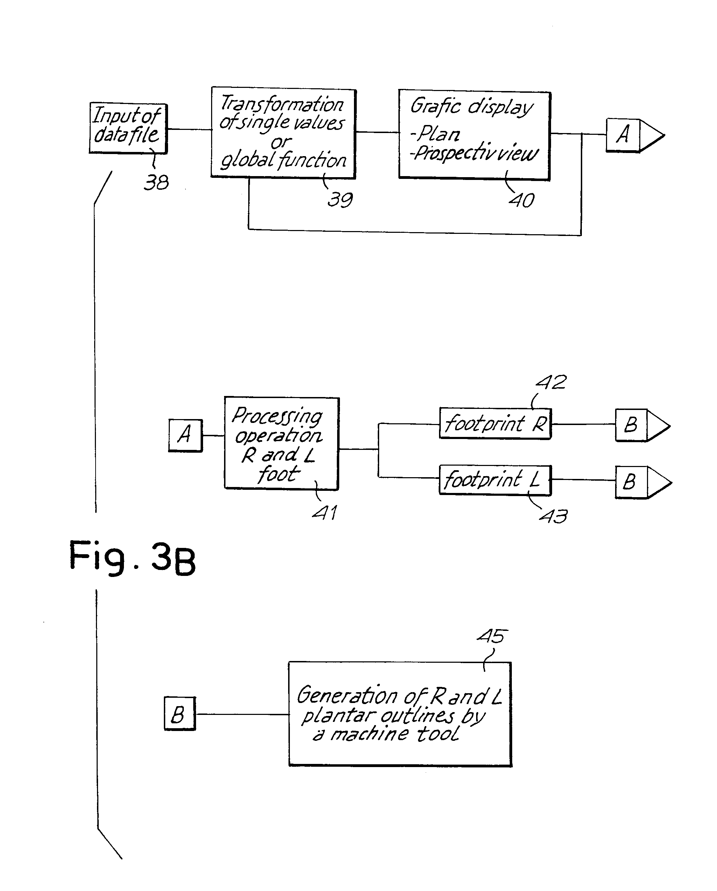

Figg. 3A and 3B show a synthetic block diagram of the production cycle according to the invention. More particularly, Fig. 3A show the blocks relevant to data acquired from the footboard (block 30), to the filtering and calibration data (block 32) and to the construction of peaks frame (block 33). The frame of maximum values is stored (block 34) together with data relevant to the foot shape and anatomy as detected by means 9, 11 or by the device shown in Fig. 2 (block 35) and with personal data entered via the keyboard (block 36). The data file thus constructed is transferred (into a magnetic support such a diskette or a tape, or via modem onto a telephone transmission or dedicated line) to the processor 23. The next processing cycle is represented by the diagram of Fig. 3B, wherein the block 38 represents the input of the data file into the processor 23, the blocks 39 and 40 represent functions involving the transformation of single values, display on a monitor or on a plotter of the plan and perspective views of the foot, the block 41 represents data processing operations for the construction of the right footprints (block 42) and of left footprints (block 43), and the block 45 represents the generation of the left and right plantar templates outlines by means of the machine tool 25.

It is understood that the drawing shows an exemplification given only as a practical demonstration of the invention, as this may vary in the forms and dispositions without nevertheless coming out from the scope of the idea on which the same invention is based.

## Claims

1. A method for making plantar templates, which comprises the steps of:
   – detecting the distribution of the foot load on the ground;
   – storing and processing data relevant to the loads distribution;
   – utilizing the precessed data to operate a numerical control machine tool which works a block of material to obtain a plantar template whose shape is determined according to the conformation of the user foot.

2. Method according to claim 1, characterized in that the static and dynamic distributions of the loads are detected.

3. Method according to claim 1 or 2, characterized in that data are determined and recorded concerning the anatomy, shape and dimension of the foot for the choice of the type of shoe wherein the plantar template will be inserted.

4. Method according to one or more of the preceding claims, characterized in that the foot-supporting area is subdivided into unit areas, and that more scannings are carried out for the acquisition of a plurality of frames of data relevant to the static and/or dynamic distribution of the loads, each frame containing the value of the unit loads on each unit area.

5. Method according to claim 4, characterized in that from the acquired data an array or frame of peak values is generated, containing, for each unit area of detection, the maximum value detected during several acquisitions carried out.

6. Method according to one or more of the preceding claims, wherein the data relevant to the load distribution and possible data relevant to the conformation and dimensions of the foot are transferred from a processor associated to the detection means to a processor associated to the machine tool for the production of the template.

7. A system for making plantar templates according to the method of one or more of the preceding claims 1 to 6, characterized in that it comprises:
   – means for detecting the distribution of the foot loads on the ground;
   – means for storing said detected data;
   – means for processing the data;
   – a numerical control machine tool, controlled by said data processing means, for working the plantar template.

8. System according to claim 7, comprising a footboard provided with piezoelectric sensors for the detection of load distribution, and an electronic processor associated to said footboard.

9. System according to claim 7 or 8, further comprising means for detecting the anatomy and/or dimensions of the foot.

10. System according to claim 7, 8 or 9, comprising a first electronic processor for detecting and storing data and a second electronic processor for controlling a machine tool according to the detected and processed data.

11. System according to claim 10, comprising a line for the transmission of data between said first and said second electronic processor.

12. Method as described and illustrated for the specified purposes.

13. System as described and illustrated for the specified purposes.

Fig. 1

# Fig. 2

# Fig.3A

Data acquisition from footboard — 30

Filtering and calibration — 32

Construction of peak frame — 33

Data acquisition from shape detection means — 35

Imput personal data — 36

Storing of data — 34

Data file transfer to machine tool processor — 37

Input of
data file

Transformation
of single values
or
global function

Grafic display
-Plan
-Prospectiv view

A

38

39

40

A

Processing
operation
R and L
foot

footprint R

B

42

footprint L

B

41

43

Fig. 3B

45

B

Generation of R and L
plantar outlines by
a machine tool

**European Patent Office**

## EUROPEAN SEARCH REPORT

Application Number

EP 92 83 0159

### DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | WO-A-8 809 147 (P. SEITZ)<br>* page 6, line 7 - page 8, line 30 *<br>--- | 1-4,6-11 | A61B5/103<br>A43D1/02 |
| X<br>X | US-A-3 696 456 (T.O. DUNHAM ET AL.)<br>* column 1, line 30 - line 46; figures 1,3 *<br>* column 2, line 18 - column 3, line 31 *<br>* column 4, line 11 - line 16 *<br>--- | 1,3,4,6<br>7,9,10 | |
| A<br>A | US-A-4 745 290 (D. FRANKEL)<br>* column 2, line 12 - line 42; figures 1-3 *<br>--- | 1,3,6,7<br>9-11 | |
| A<br>A | US-A-4 454 618 (B.O. CURCHOD)<br>* column 3, line 1 - line 45 *<br>* column 5, line 4 - line 39 *<br>* column 7, line 21 - column 8, line 19 *<br><br>----- | 1,3,6,7<br>9,10 | |

| | | | TECHNICAL FIELDS SEARCHED (Int. Cl.5) |
|---|---|---|---|
| | | | A61B<br>A43D |

The present search report has been drawn up for all claims

| Place of search | Date of completion of the search | Examiner |
|---|---|---|
| THE HAGUE | 02 JULY 1992 | RIEB K.D. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
........................................................................
& : member of the same patent family, corresponding document

EPO FORM 1503 03.82 (P0401)